Europäisches Patentamt

European Patent Office

Office européen des brevets

(11) Publication number: **0 450 263 A2**

# EUROPEAN PATENT APPLICATION

(21) Application number: 91100017.2

(51) Int. Cl.⁵: **C08B 37/00, C12N 9/26**

(22) Date of filing: 02.01.91

(30) Priority: 06.04.90 JP 90281/90

(43) Date of publication of application:
09.10.91 Bulletin 91/41

(84) Designated Contracting States:
DE FR GB IT SE

(71) Applicant: Kyodo Milk Industry Corporation
Limited
17-2, Koami-cho Nihonbashi
Chuo-ku Tokyo(JP)

(72) Inventor: Oishi, Hifumi
No.303, 27-6, Sekimachi-higashi 1-chome
Nerima-ku, Tokyo(JP)
Inventor: Hattori, Takashi
No.205, 1-19, Jyosuihoncho 5-chome
Kodaira-shi, Tokyo(JP)
Inventor: Watanabe, Masatoshi
5-36, Midoricho 2-chome
Koganei-shi, Tokyo(JP)
Inventor: Kato, Akio
24-28, Kitamachi 4-chome
Kokubunji-shi, Tokyo(JP)

(74) Representative: Lesser, Karl-Bolko, Dipl.-Ing.
Patentanwalt & European Patent Attorney
Gnesener Strasse 2
W-8068 Pfaffenhofen 1(DE)

(54) Alpha-amylase inhibitor obtained from soy-bean and production process therefore.

(57) An α-amylase inhibitor which is a polysaccharide obtained from soy-bean and having a molecular weight of approximately 1.200 by gel filtration method. The α-amylase inhibitor is produced in a simplified method of using a step of removing proteins in soy-bean with ammonium sulfate or the like and then removing substances with a molecular weight of between 4.000 and 1.000.

EP 0 450 263 A2

Background of the invention

Field of the invention

The present invention concerns with an α-amylase inhibitor obtained from soy-bean and a production process therefore.

Description of the prior art

Seeds in plants contain enzymes together with inhibitors specific to each of them. Among all, detailed studies have been made on soy-bean for such inhibitors, including α-amylase inhibitors.

Production processes for α-amylase inhibitors reported so far are extremely complicate and suffer from various restrictions such as cost or yield when they are put to industrial use. Accordingly, the present inventors have tried to simplify the production process for α-amylase inhibitors. Through the study, the inventors have discovered non-protein α-amylase inhibitors not known so far, identified the substance and originated an extremely simple production process and have accomplished the present invention.

Summary of the invention

The feature of the present invention resides in an α-amylase inhibitor which is a polysaccharide obtained from soy-bean and having a molecular weight of about 1.200 (by gel filtration method).

Another feature of the present invention resides in a process for producing an α-amylase inhibitor which comprises using a step of removing proteins in soy-bean by means of ammonium sulfate or the like and then removing substances with a molecular weight of between 4.000 and 1.000.

Example

(1) Production process

Soy-bean was immersed in a twice volume of deionized water for one night and pulverized by a pulverizer such as Masukoroyder to prepare a soy-bean milk. Then, ammonium sulfate was added to a saturated concentration of 50 - 60 % and, subsequently, precipitates were removed by centrifugation or filtration. The resultant solution was subjected to ultra-filtration by using a filter or module with the molecular size of 4.000. The filtrate was further concentrated and desalted by using a filter or module with the molecular size of less than 1.000 or reverse osmosis (RO) and then lyophilized to obtain an α-amylase inhibitor. The yield was 0,75 g per 1 g of the whole soy-bean.

(2) α-amylase inhibitor activity

The activity of the α-amylase inhibitor was determined in accordance with each of a CM-amylose-DEX method and an iodine starch method. This is, after preincubating an α-amylase inhibitor obtained from soy-bean and α-amylase derived from human saliva at 37 °C or 30 min, a substrate (CM-amylose or starch) was added and brought into reaction at 37 °C for 10 min. One unit of the α-amylase inhibitor was determined as a activity factor for 50 % inhibition of one unit of α-amylase.

The results are shown in Table 1.

Table 1: Effect of α-amylase inhibitor on each α-amylase activity.

| | α-Amylases | | | |
|---|---|---|---|---|
| | Micro-organism | Human saliva | Human pancreatic juice | Mouse pancreatic juice |
| Activity of α-amylase inhibitor (unit) | 18.000 | 9.180 | 48.900 | 47.040 |

(3) α-amylase inhibitor activity against amylopectine as substrate

That is, amylopectine derived from corn and potato (Sigma Co.) was used as the substrate (6,0 mg/ml, Tris-HCl buffer saline, TBS), α-amylase derived from human saliva (Sigma Co.), α-amylase derived from Bacillus subtilis, α-amylase derived from mouse pancreas (Sigma Go.) and β-amylase derived from Bacillus (Sigma Co.) were dissolved in TBS (1 unit/ml). α-amylase inhibitor was dissolved also in an identical buffer (1 mg/ml). After preincubating 500 $\mu$l of α- or β-amylase at 37 °C for 10 min, 100 $\mu$l of amylopectine was added and reacted for one hour at the same temperature. Then, dinitrosalycillic acid was added and the amount of the resultant maltose was measured. The results are shown in Table 2.

Table 2: Variation of α-amylase inhibitor activity against amylopectine as substrate

| Origin of amylopectine | Origin of α- and β-amylase | | | |
|---|---|---|---|---|
| | Human saliva | Bacillus | Mouse pancreas | Bacillus |
| Potato | 70* | 60* | 60* | 20* |
| Corn | 80* | 50* | 74* | 30* |

Amylopectine was used as the substrate of α-amylases.

*: 1 unit of α-amylase inhibitor obtained from soy-bean was acted on 1 unit of each enzyme and the inhibitor activity was expressed as percentage.

(4) Property of α-amylase inhibitor

Molecular weight was determined by gel filtration method. That is, the α-amylase inhibitor was dissolved in O,1M Tris-HCl (pH 7,2), loaded to a high performance liquid chromatography with gel filtration column (Asahipack GS-520) equilibrated with the same buffer and then eluted by the same buffer. As the molecular weight standard, polyethylene glycol (molecular weight 20 K, 7,5 K, 3,0 K and 0,6 K) was used.

3

The total saccharide content in this substance was measured based on a phenol-sulfate method. Constituent monosaccharide ingredient were analyzed by hydrolyzing the substance and then using a NH$_2$ type reverse phase column and 85 % acetonitrile (mobile phase solvent). Further, hexosamine was analysed by using an amino acid analyzer. The results are shown in Table 3a.

## Table 3a: Property of α-amylase inhibitor

| | |
|---|---|
| Molecular weight | 1.200 |
| Constituent saccharide | Arabinose, Glucose, Galactose, Galactosamine, Glucosamine |
| Heat stability (0,1 % solution) | 75 °C for 60 min and 25 °C for 10 days |

Heat stability was measured as shown below. That is, the α-amylase inhibitor was dissolved in a deionized water and left under each of temperature conditions, for 60 min for the former case and 10 days in the latter case, and residual activity of the α-amylase inhibitor was measured. The results are shown in Tables 3b and 3c.

## Table 3b: Heat stability of the α-amylase inhibitor at 75 °C

| | Processing time (min) | | | | |
|---|---|---|---|---|---|
| | 0 | 5 | 10 | 30 | 60 |
| Residual activity (%) | 100 | 99,4 | 98,6 | 99,7 | 99,2 |

## Table 3c: Heat stability of the α-amylase inhibitor at 25 °C

| | Processing time (day) | | | | |
|---|---|---|---|---|---|
| | 0 | 2 | 6 | 8 | 10 |
| Residual activity (%) | 100 | 99,7 | 99,2 | 98,5 | 94,8 |

## Claims

1. An α-amylase inhibitor which is a polysaccharide obtained from soy-bean and having a molecular weight of approximately 1.200 by gel filtration method.

2. An α-amylase inhibitor containing about 70% of saccharide the constituent monosaccharide ingredients of which are arabinose, gluoose, galactose, glucosamine and galactosamine.

3. An α-amylase inhibitor having a stability to a heat treatment at 75 °C for 60 min and 25 °C for 10 days.

4. A process for producing an α-amylase inhibitor which comprises using a step of removing proteins in soy-bean by ammonium sulfate or the like and then removing substances with a molecular weight of between 4.000 and 1.000.